# EUROPEAN PATENT SPECIFICATION

(11) **EP 2 441 380 B1**
(45) Date of publication and mention of the grant of the patent: **05.10.2016**
(21) Application number: 10785881.3
(22) Date of filing: 19.04.2010
(51) Int. Cl.: A61B 1/04, H04N 7/18, H04W 88/02, H04W 72/02, H04W 72/08, A61B 1/00, H04W 84/12

(54) **WIRELESS ENDOSCOPIC APPARATUS, RECEIVING DEVICE THEREOF, AND RECEIVING METHOD**
DRAHTLOSE ENDOSKOPVORRICHTUNG, EMPFANGSGERÄT DAFÜR UND EMPFANGSVERFAHREN
APPAREIL ENDOSCOPIQUE SANS FIL, DISPOSITIF DE RÉCEPTION AFFÉRENT ET PROCÉDÉ DE RÉCEPTION

(30) Priority: 10.06.2009 JP 2009139420
(43) Date of publication of application: 18.04.2012
(73) Proprietor: Olympus Corporation, Tokyo 192-8507 (JP)
(72) Inventor: ENDO, Takahisa, Tokyo 151-0072 (JP); HONDA, Takemitsu, Tokyo 151-0072 (JP)
(74) Representative: Gunzelmann, Rainer
(86) International application number: PCT/JP2010/002821
(87) International publication number: WO 2010/143349

(56) References cited:
- EP-A1- 2 493 193
- EP-A2- 1 220 499
- EP-A2- 1 707 106
- JP-A- 2 244 828
- JP-A- 4 341 024
- JP-A- 9 261 747
- JP-A- 10 285 629
- JP-A- 2001 353 124
- JP-A- 2002 335 191
- JP-A- 2003 264 872
- JP-A- 2005 033 338
- JP-A- 2005 101 787
- JP-A- 2006 271 432
- JP-A- 2006 271 433
- JP-A- 2007 097 653
- JP-A- 2008 036 318
- JP-A- 2008 042 896
- JP-A- 2008 148 215
- US-A1- 2004 090 929
- US-A1- 2004 196 364
- US-A1- 2005 152 299

## Description

### TECHNICAL FIELD

The present invention relates to a wireless endoscopic apparatus, a receiving device thereof, and a receiving method.

### BACKGROUND ART

In recent years, endoscopic apparatuses that enable a subject image of an inside of a body cavity or a conduit to be observed by inserting an elongated insert member into the body cavity or the conduit have been widely used. In general, the above-described endoscopic apparatus includes an endoscope having the insert member to be inserted into the body cavity or the conduit and a main body device having a light source device or a video processor. The endoscope and the main body device are connected by a light guide cable, which guides illumination light from the light source device to the endoscope, and a signal cable, which transmits an image pickup signal obtained by the endoscope to the video processor. Thereby, a moving range of the endoscope is limited and the operability of the endoscope is hindered.

For example, in Patent Document 1, an illumination device constituted by a light emitting diode (LED) and the like is embedded in the endoscope, so that the light guide cable extending from the endoscope is removed. A video signal processing circuit, which obtains a video signal capable of being displayed on a monitor by performing video signal processing of an image pickup signal, and a transmitting circuit, which transmits the video signal by an radio wave, are provided in the endoscope, and a receiving device, which receives the radio wave and demodulates the video signal, is provided separately from the endoscope so that the signal cable extending from the endoscope is removed. In general, the above-described endoscopic apparatus is also referred to as a wireless endoscopic apparatus, and has an advantage in that the limitation of a moving range of the endoscope is mitigated and the operability thereof is improved.

### CITATION LIST

Patent Document 1: Japanese Unexamined Patent Application, First Publication, No. S60-48011

Document EP 1 707 106 A2 discloses an electronic endoscope and a processor of an electronic endoscope apparatus which transmit and receive signals via radio waves within a predetermined frequency band to which plural channels are allocated. The electronic endoscope has a transmission frequency band switching circuit which switches between a first and second transmission frequency bands, and a first channel detection circuit for detecting an available channel. The processor has a reception frequency band switching circuit which switches between first and second reception frequency bands, and a second channel detection circuit for detecting an available channel. The transmission and reception frequency band switching circuits automatically switch the transmission and reception frequency bands according to detection results of the first and second channel detection circuits.

### SUMMARY OF INVENTION

### Problems to be Solved by the Invention

Because the receiving device is provided separately from the endoscope in the wireless endoscopic apparatus, it is necessary to set up a communication channel of the endoscope of a transmitting side in accordance with a communication channel set in the receiving device and establish a connection of wireless communication. A method of uniquely determining a combination of the receiving device and the endoscope and fixedly pre-setting a communication channel to an arbitrary communication channel is also possible. However, because disinfection/sterilization treatments and examinations of the endoscopes are simultaneously processed in a hospital using a plurality of receiving devices and a plurality of endoscopes, the combination of the receiving device and the endoscope is not uniquely determined. In order to prevent the interference of radio waves, it is necessary to set communication channels of the receiving devices to be different from each other.

Thus, a switch for selecting a communication channel is provided in each of the receiving device and the endoscope, and the same communication channel as the communication channel set in the receiving device of an object to be used at the initiation of use of the endoscopic apparatus is set in the endoscope of an object to be used, so that a wireless communication connection between the receiving device and the endoscope is established.

In data communication to be performed by a general personal computer via a wireless LAN, a connection to the wireless LAN is established by a method of selecting a wireless terminal (AP) to be connected by a user after detecting a connectable wireless terminal (AP) by searching for all available communication channels. In recent years, a function of searching for all communication channels available in a wireless LAN at power-up, detecting empty communication channels unused by other wireless terminals, and automatically setting up a communication channel from the empty communication channels has been provided in a household wireless LAN AP. As described above, because a communication channel to be connected is determined in the wireless LAN of a general personal computer after all available communication channels are searched for, it takes time for the connection to be established.

On the other hand, efficiently selecting a communication channel having a good communication state is a goal to improve the operability in a communication connection of the endoscopic apparatus.

The present invention has been made in view of the above-described problems, and an object of the invention is to provide a wireless endoscopic apparatus, a receiving device thereof, and a receiving method capable of efficiently selecting a communication channel of which a communication state is good.

### Means for Solving the Problems

According to the present invention for solving the above-described problem, there is provided a receiving device of a wireless endoscopic apparatus including the features of claim 1.

The receiving device of the wireless endoscopic apparatus of the present invention may further include: an output unit for outputting information for prompting selection of a communication channel group different from the selected communication channel group if the determination unit determines not to perform the communication.

In the receiving device of the wireless endoscopic apparatus of the present invention, the output unit may output information regarding a use state of a communication channel to perform the communication if the determination unit determines to perform the communication.

The receiving device of the wireless endoscopic apparatus of the present invention may further include: a operation unit for allowing an operator to select a communication channel group and transferring a selection result to the determination unit.

In the receiving device of the wireless endoscopic apparatus of the present invention, the determination unit may detect communication traffic of a communication channel belonging to the selected communication channel group as the use state.

In the receiving device of the wireless endoscopic apparatus of the present invention, the determination unit may determine whether or not to perform communication using the communication channel belonging to the selected communication channel group on the basis of presence of a communication channel of which the communication traffic is less than a predetermined threshold.

In the receiving device of the wireless endoscopic apparatus of the present invention, the determination unit may determine whether or not to perform communication using the communication channel belonging to the selected communication channel group on the basis of presence of a communication channel of which the received signal strength is less than a predetermined threshold.

In the receiving device of the wireless endoscopic apparatus of the present invention, the determination unit may determine to perform communication using a communication channel belonging to a communication channel group having a smallest total of communication traffic of communication channels belonging to the same communication channel group.

In the receiving device of the wireless endoscopic apparatus of the present invention, the storage unit may store a communication channel group to which a communication channel having a use frequency band not overlapping that of a specific communication channel belonging to which another communication channel group belongs.

In the receiving device of the wireless endoscopic apparatus of the present invention, the storage unit may store a communication channel group to which a communication channel having an overlap of a use frequency band with that of a specific communication channel belonging to the same communication channel group belongs, wherein the overlap is equal to or greater than a predetermined range.

According to the present invention, there is provided a receiving method for use in a receiving device of a wireless endoscopic apparatus, including the steps of claim 11.

A wireless endoscopic apparatus includes the features of claim 12.

### Effect of the Invention

According to the present invention, it is possible to efficiently select a communication channel of which a communication state is good by detecting use states of communication channels belonging to a selected communication channel group and determining whether or not to perform communication using a communication channel belonging to the selected communication channel group on the basis of a detection result, as compared to the case where all communication channels are searched for.

### BRIEF DESCRIPTION OF THE DRAWINGS

FIG. 1 is a configuration diagram showing a configuration of an endoscopic apparatus according to an embodiment of the present invention.
FIG. 2 is an external appearance diagram of an endoscope according to an embodiment of the present invention.
FIG. 3 is a block diagram showing a configuration of the endoscope according to an embodiment of the present invention.
FIG. 4 is a block diagram showing a configuration of a receiving device according to an embodiment of the present invention.
FIG. 5 is a flowchart showing an operation of the receiving device according to an embodiment of the present invention.
FIG. 6 is a flowchart showing an operation of the receiving device according to an embodiment of the present invention.
FIG. 7 is a flowchart showing an operation of a logical connection according to an embodiment of the present invention.
FIG. 8 is a flowchart showing an operation of the endoscope according to an embodiment of the present invention.
FIG. 9 is a flowchart showing an operation of the receiving device according to an embodiment of the present invention.
FIG. 10 is a flowchart showing an operation of the receiving device according to an embodiment of the present invention.
FIG 11 is a flowchart showing an operation of the receiving device according to an embodiment of the present invention.
FIG. 12 is a reference diagram showing content of a communication channel setting table according to an embodiment of the present invention.
FIG. 13 is a reference diagram showing content of a search table according to an embodiment of the present invention.
FIG. 14 is a reference diagram showing content of a communication channel setting table according to an embodiment of the present invention.
FIG. 15 is a reference diagram showing content of a search table according to an embodiment of the present invention.
FIG. 16 is a reference diagram showing a frequency band in which a communication channel is used.
FIG. 17 is a reference diagram showing a frequency band in which a communication channel is used.
FIG. 18 is a reference diagram showing a frequency band in which a communication channel is used.

### BEST MODE FOR CARRYING OUT THE INVENTION

Hereinafter, the present invention will be described with reference to specific embodiments.

Hereinafter, the embodiments of the present invention will be described with reference to the drawings. As described above, to improve operability in a communication connection of an endoscopic apparatus, efficiently selecting a communication channel having a good communication state is a goal. Another goal is to implement power saving by shortening the connection waiting time of an endoscope driven by a battery. As described below, there is a problem in that a communication error occurs due to a frame collision in the endoscopic apparatus.

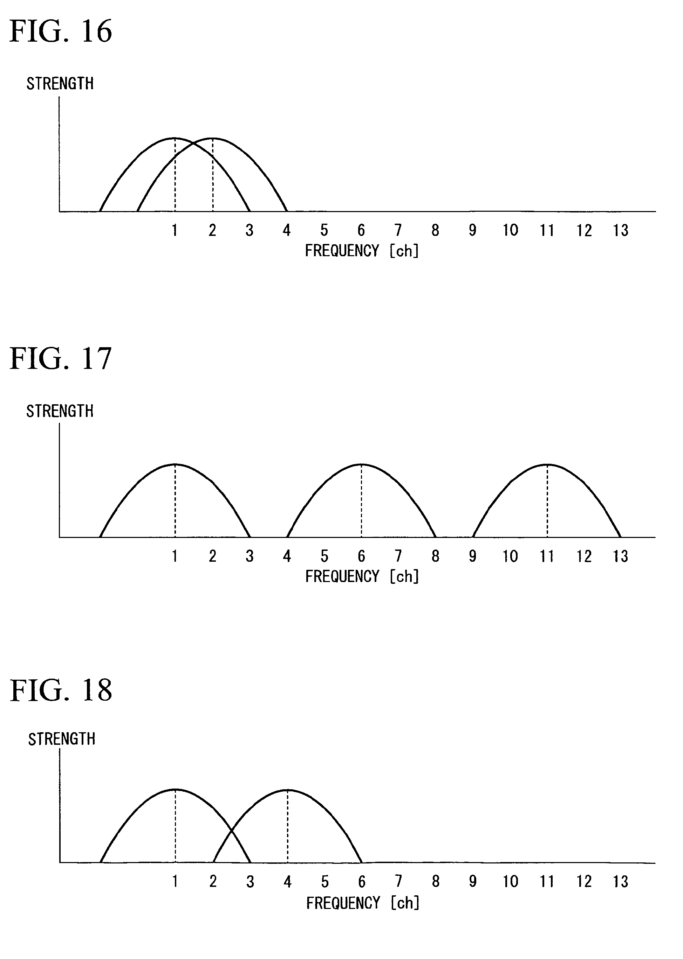

As a communication system to be used in wireless communication, it is effective to use a wireless communication system such as IEEE 802.11 used in a wireless LAN in which high-speed data communication is possible. In the wireless communication system, the wireless communication is performed by selecting any communication channel from a plurality of communication channels so as to effectively use a frequency band. As shown in FIG. 16, each communication channel is arranged to have a use frequency band of which a part thereof overlaps that of another communication channel due to a limit of an available frequency band.

In data communication within a set communication channel, a process of avoiding a collision between transmission frames is performed by control of IEEE 802.11 protocol. However, if another wireless terminal performs data communication by a communication channel adjacent to the set communication channel, the process of avoiding a collision with a transmission frame of an adjacent communication channel does not work because the endoscopic apparatus does not recognize the transmission frame of the adjacent communication channel.

Thus, there is a problem in that a frame collision occurs if a transmission frame from the endoscope and a transmission frame from a wireless terminal using a communication channel adjacent to a communication channel used by the endoscope are transmitted at the same timing. If a radio wave output of the wireless terminal is large, there is a problem in that the receiving device does not normally receive image data transmitted from the endoscope and hence an image thereof is interrupted.

In data communication performed by a general personal computer via the wireless LAN, the occurrence of a communication error does not become a large problem when a data arrival by retransmission is ensured because a delay of data communication is allowed to a certain extent. In order to cause a large number of wireless LANs to co-exist, it is necessary to efficiently allocate a communication channel rather than to avoid the occurrence of a communication error due to a temporary frame collision. A communication channel to be used is determined on the basis of a determination of only whether or not the communication channel to be used is used in other communication terminals.

On the other hand, in the endoscopic apparatus, it is important to transmit/receive all image data of a communication object without loss or delay so as to ensure operability and it is necessary to perform wireless communication at a low communication error rate by selecting a communication channel having a good wireless environment. Thus, the occurrence of a communication error due to a frame collision with a communication channel adjacent to a communication channel used by the endoscopic apparatus is problematic.

In view of the above, the endoscopic apparatus (wireless endoscopic apparatus) according to this embodiment implements power saving and reduces the occurrence of a communication error by efficiently selecting a communication channel having a good communication state. In this embodiment, it is assumed that IEEE 802.11 is used as an example of the wireless communication system.

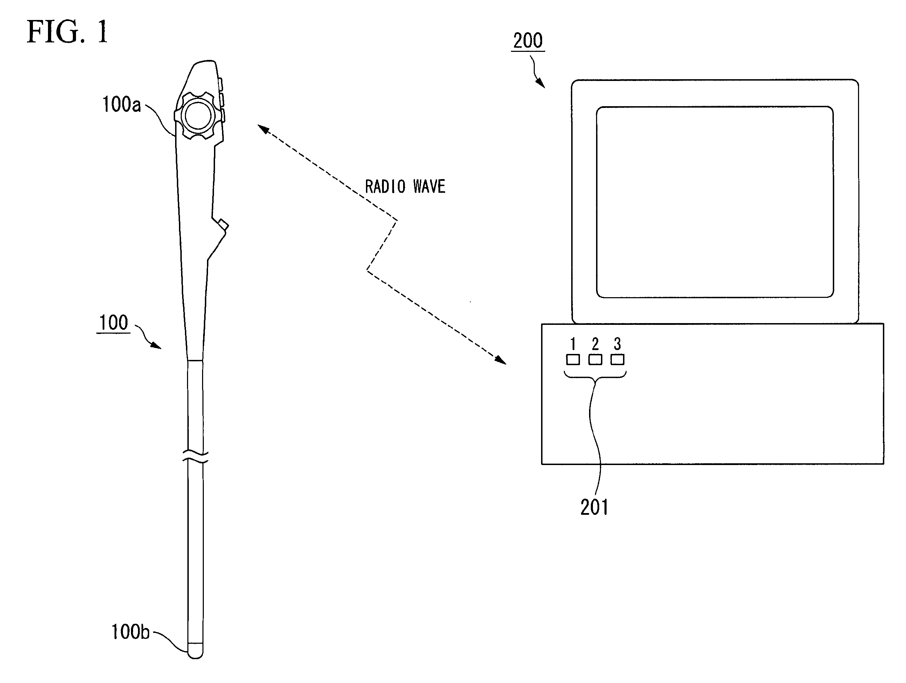

FIG. 1 shows a configuration of an endoscopic apparatus according to this embodiment. The endoscopic apparatus includes an endoscope 100, which transmits captured image data by wireless communication, and a receiving device 200, which receives the image data transmitted from the endoscope 100 and displays an image on a monitor. The endoscope 100 includes an operation unit 100a including a plurality of switches for allowing an operator to input an operating instruction. The receiving device 200 includes a communication setting display unit 201 including a plurality of LEDs, which indicate a communication setting state of the receiving device 200. Although not shown in FIG. 1, a CH (channel) setting switch is mounted on the back of the receiving device 200.

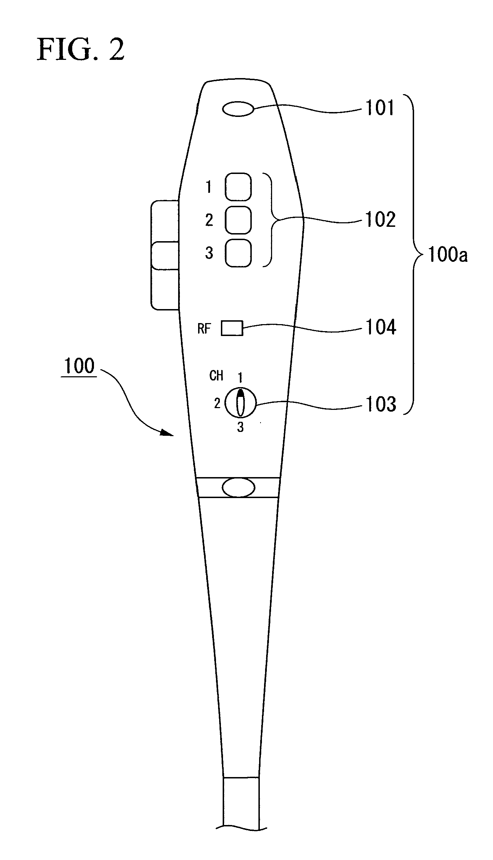

FIG. 2 shows a state of the endoscope 100 when viewed from a layout surface of operating switches. An operation unit 100a of the endoscope 100 includes a power switch 101, a plurality of operating switches 102, a CH setting switch 103, and a state indication LED 104. A number for identifying a set CH is assigned to the CH setting switch 103.

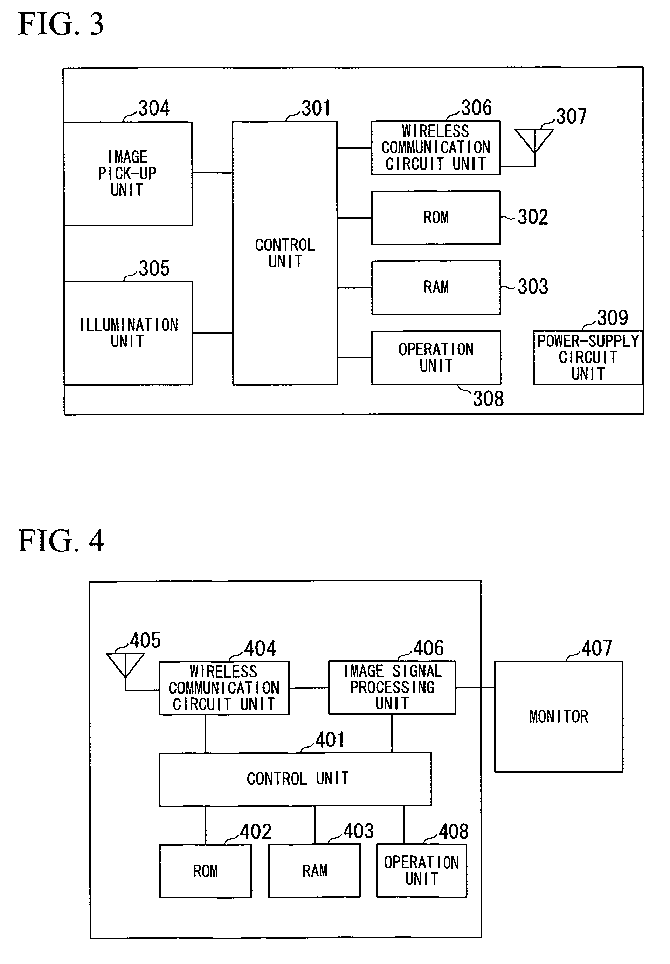

FIG. 3 shows an electrical configuration of the endoscope 100. The endoscope 100 includes a control unit 301, a ROM 302, a RAM 303, an image pickup unit 304, an illumination unit 305, a wireless communication circuit unit 306, an antenna 307, an operation unit 308, and a power-supply circuit unit 309.

The control unit 301 operates according to a program stored in the ROM 302, and controls an operation sequence of the endoscope 100. The ROM 302 is a non-volatile memory such as a flash ROM. Program data for controlling the endoscope 100 and various setting information including communication setting parameters are stored in the ROM 302. The communication setting parameters include a communication channel (frequency), a service set identifier (SSID), wired equivalent privacy (WEP), and the like corresponding to each number assigned to the CH setting switch 103.

The RAM 303 is used as a buffer, which temporarily buffers image data output from the image pickup unit 304, a work area to be used in an arithmetic operation and the like of the control unit 301, and an area where various settings and the like are temporarily stored.

The image pickup unit 304 includes a lens, which forms an image of incident light, a photoelectric converter (a CCD or CMOS sensor, or the like), which converts light of which an image is formed into an electric signal, an analog-to-digital (AD) converter, which converts an analog electric signal output from the photoelectric converter into a digital electric signal, and the like.

The illumination unit 305 includes an irradiation lens, an LED, an LED drive circuit, and the like, and is arranged on a tip end 100b (FIG. 1) of the endoscope 100. Light emitted from the LED is irradiated to a body to be observed within a body cavity via the irradiation lens. The illumination unit 305 may have a configuration that the LED is arranged inside the operation unit 100a, not on the tip end 100b, and light is guided to the tip end 100b by a light guide.

The wireless communication circuit unit 306 includes a high-frequency circuit unit necessary for wireless communication, an encoding/decoding circuit unit, a buffer memory, and the like, and is connected to the antenna 307. To perform wireless communication with the receiving device 200, it is necessary to set the CH setting switch 103 to the same number as that of a CH setting switch set in the receiving device 200 so as to set the same communication channel, SSID, and the like as those set in the receiving device 200. Communication setting parameters corresponding to a communication channel designated by each number of the CH setting switch 103 are set in the wireless communication circuit unit 306.

The operation unit 308 (corresponding to the operation unit 100a of FIG. 1) has the power switch 101, the operating switch 102, and the CH setting switch 103 shown in FIG. 2, and outputs states and state variations of buttons and switches as electric signals. The state indication LED 104 for providing notification of a state of a connection to the receiving device 200 is arranged in the operation unit 308.

The power-supply circuit unit 309 includes a battery, a DC/DC converter, and the like, and supplies power to each block described above by sensing the turn-on of the power switch 101.

FIG. 4 shows an electrical configuration of the receiving device 200. The receiving device 200 includes a control unit 401, a ROM 402, a RAM 403, a wireless communication circuit unit 404, an antenna 405, an image signal processing unit 406, a monitor 407, and a operation unit 408.

The control unit 401 operates according to a program stored in the ROM 402, and controls an operation sequence of the receiving device 200. The ROM 402 is a non-volatile memory of a flash ROM or the like. Program data for controlling the receiving device 200, various setting information including communication setting parameters, a communication channel setting table, and a search table are stored in the ROM 402.

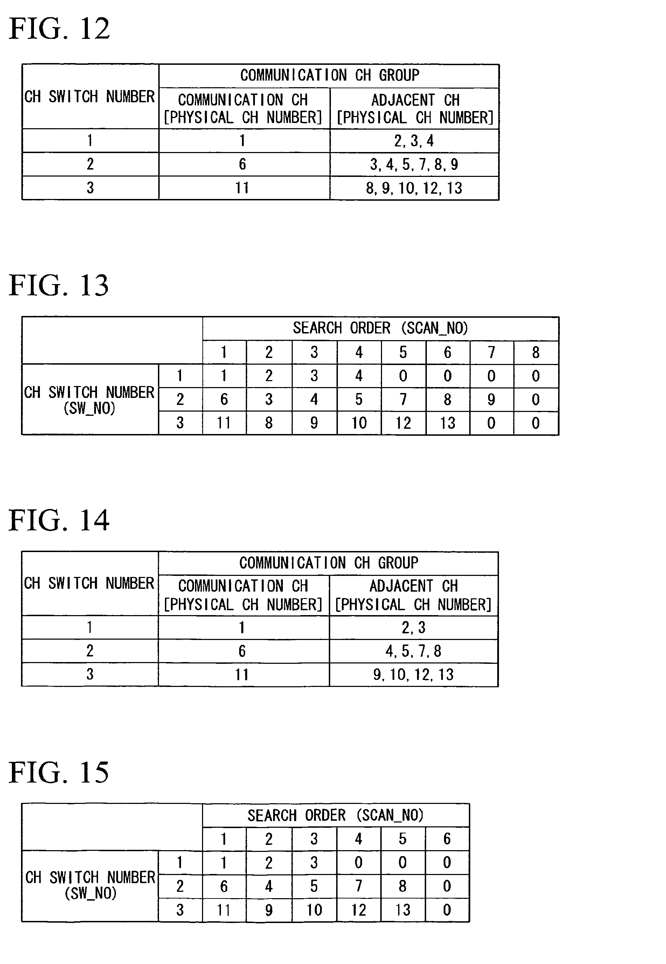

FIG. 12 shows content of the communication channel setting table. In the communication channel setting table, a communication channel number is associated with each number of a CH setting switch (a CH switch number). In IEEE 802.11, it is possible to perform wireless communication by selecting any communication channel from a plurality of communication channels. Although center frequencies of communication channels are separated by 5 MHz from each other, an overlap of a use frequency band occurs between adjacent communication channels as shown in FIG. 16 because each communication channel uses a frequency band of about 20 MHz.

In this embodiment, 13 communication channels are provided. Each of the 13 communication channels belongs to at least one of 3 communication channel groups corresponding to CH switch numbers. For example, a communication channel group including communication channels 1, 2, 3, and 4 corresponds to a CH switch number 1, a communication channel group including communication channels 3, 4, 5, 6, 7, 8, and 9 corresponds to a CH switch number 2, and a communication channel group including communication channels 8, 9, 10, 11, 12, and 13 corresponds to a CH switch number 3.

One communication channel of the communication channels belonging to each communication channel group is used in a logical connection to be described later. For example, if the CH switch number 1 is set, the communication channel 1 is used in the logical connection. If the CH switch number 2 is set, the communication channel 6 is used in the logical connection. If the CH switch number 3 is set, the communication channel 11 is used in the logical connection. The communication channels 1, 6, and 11 are the same as communication channels set by the CH setting switch 103 in the endoscope 100. As shown in FIG. 17, frequency bands of the communication channels 1, 6, and 11 do not overlap. One communication channel group includes one communication channel (written as a communication channel in FIG. 12) used in the logical connection and communication channels (written as adjacent CHs in FIG. 12) each having a use frequency band partially overlapping that of the one communication channel.

When the above is more generalized, n (n>1) communication channels each having a use frequency band partially overlapping that of at least one other communication channel are provided, and x (1<x≤) communication channel groups to which L (1≤L<n) communication channels belong are provided. In an example shown in this embodiment, n=13, L=4 (the CH switch number 1), 7 (the CH switch number 2), and 6 (the CH switch number 3), and x=3.

FIG. 13 shows content of the search table. In the search table, a search order and a communication channel number belonging to each communication channel are associated with each CH switch number. Although there is the search order in which 0 is stored as the communication channel number, 0 is a value used to determine an end of a search phase to be described later. In the search phase, a use state of a communication channel is detected in order according to the search order.

Returning to the description of a configuration shown in FIG. 4, the RAM 403 is used as a buffer, which temporarily buffers image data received by the wireless communication circuit unit 404, a work area to be used in an arithmetic operation and the like of the control unit 401, and an area where various settings and the like are temporarily stored.

The wireless communication circuit unit 404 includes a high-frequency circuit unit necessary for wireless communication, an encoding/decoding circuit unit, a buffer memory, and the like, and is connected to the antenna 405. Like the wireless communication circuit unit 306 of the endoscope 100, the wireless communication circuit unit 404 performs wireless communication according to a protocol of the wireless LAN. A communication channel designated by a CH switch number set by the CH setting switch is read from the communication channel setting table of FIG. 12, and communication setting parameters corresponding to the communication channel are set in the wireless communication circuit unit 404.

The image signal processing unit 406 converts image data received by the wireless communication circuit unit 404 into a National Television System Committee (NTSC) signal or a Phase Alternating Line (PAL) signal, and outputs it to the monitor 407. The monitor 407 includes a liquid crystal display (LCD) device and its control circuit, displays an image, and operates as a notification unit for providing notification of a state of a wireless connection.

Although not shown in FIG. 1, the operation unit 408 has a CH setting switch mounted on the backside of the receiving device 200, and outputs a state and a state variation of the CH setting switch as an electric signal. Also, the communication setting display unit 201 (FIG. 1), which indicates a communication channel selected by the CH setting switch by an LED, is arranged in the operation unit 408.

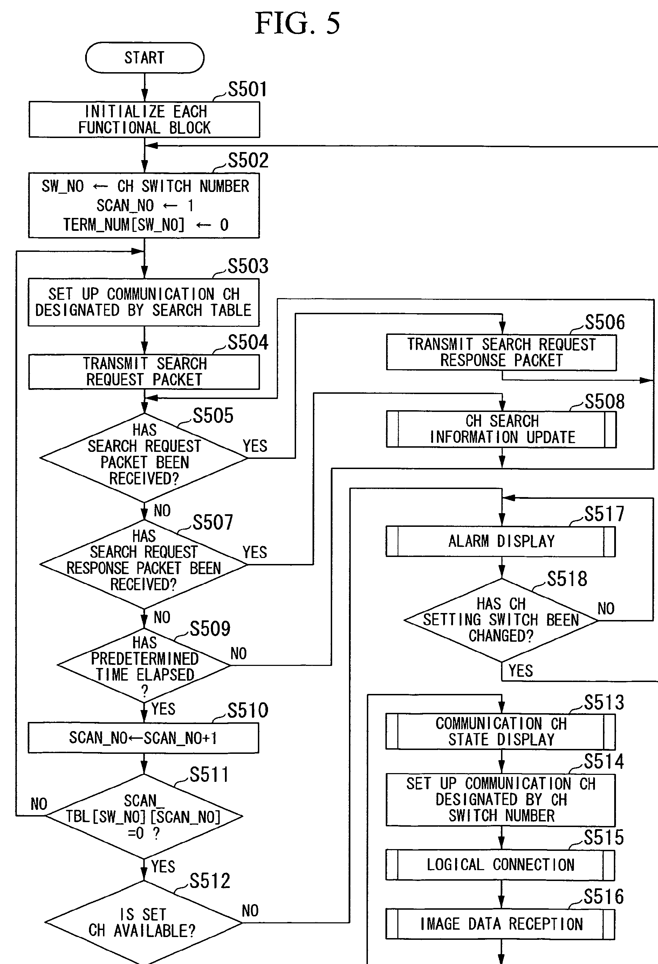

Next, an operation of the endoscopic apparatus according to this embodiment will be described. Hereinafter, four operation examples will be described. First, a first operation example will be described. Hereinafter, an operation of the receiving device 200 will be described according to FIG. 5. In this embodiment, it is assumed that the operator turns on power of the endoscope 100 to be used after the power of the receiving device 200 to be used is turned on.

The operator turns on the power of the receiving device 200 after setting a communication channel by the CH setting switch of the receiving device 200. If the receiving device 200 is powered on, the control unit 401 initializes each functional block of the receiving device 200 (step S501). At this time, an LED corresponding to a set communication channel among the LEDs of the communication setting display unit 201 is turned on to indicate communication setting set by the CH setting switch of the receiving device 200.

Subsequently, the control unit 401 initializes parameters SW_NO, SCAN_NO, and TERM_NUM[SW_NO] to be later used in control (step S502). SW_NO is a parameter for storing a CH switch number, and is stored as a CH switch number set by the CH setting switch during initialization. SCAN_NO is a parameter for storing a search order in the search table, and is stored as 1 during the initialization. TERM_NUM[SW_NO] is a parameter for storing the number of peripheral wireless communication terminals using a communication channel satisfying a predetermined condition, and is stored as 0 during the initialization. TERM_NUM[SW_NO] includes three values of TERM_NUM[1], TERM_NUM[2], and TERM_NUM[3].

Subsequently, the receiving device 200 establishes a physical connection of wireless communication by a communication channel selected by the operator as follows. In the physical connection, a radio frequency and an SSID to be used in a connection in a physical layer are determined, and a state is reached in which a packet to be transmitted/received to/from a communication partner is input to hardware. First, the receiving device 200 performs the transition to a search phase of a communication channel.

Upon transition to the search phase, the control unit 401 reads a number of a communication channel (a communication channel for which the CH switch number is SW_NO and the search order is SCAN_NO) designated by SW_NO and SCAN-NO from the search table, reads communication setting parameters corresponding to the communication channel from the ROM 402, and sets the communication parameters to the wireless communication circuit unit 404 (step S503). In a communication channel search, a search request packet is transmitted and a search request response packet to the search request packet is received during a predetermined period. Thus, according to the invention, the control unit 401 causes the wireless communication circuit unit 404 to transmit the search request packet in a broadcast scheme (step S504). A communication terminal in which the same communication channel as that of the receiving device 200 is set receives the search request packet transmitted from the receiving device 200, and transmits the search request response packet.

After transmission of the search request packet, the control unit 401 determines whether or not a search request packet from another wireless communication terminal has been received (step S505). If the search request packet has been received, the control unit 401 causes the wireless communication circuit unit 404 to transmit a search request response packet in a unicast scheme (step S506). Thereafter, the process returns to step S505. If the search request packet has not been received, the control unit 401 determines, according to the invention, whether or not a search request response packet from another wireless communication terminal has been received (step S507). If the search request response packet has been received, the control unit 401 executes, according to the invention, a process of updating the value of TERM_NUM[SW_NO] (CH search information) (step S508). Thereafter, the process returns to step S505. If the search request response packet has not been received, the process moves to step S509.

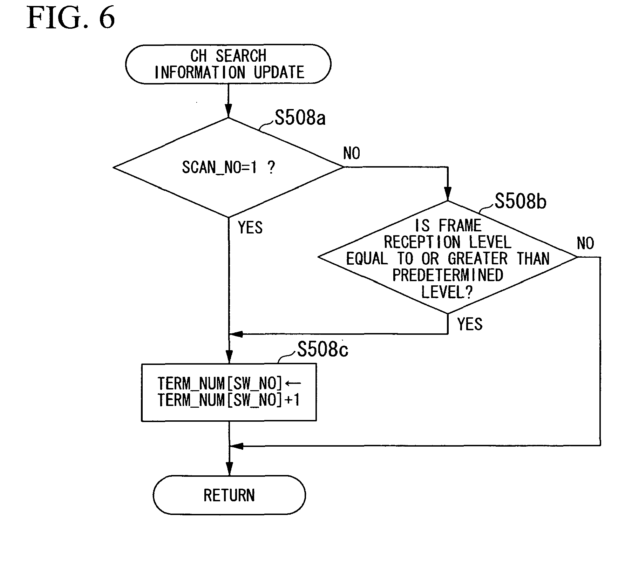

FIG. 6 shows details of step S508 which form part of the invention. The control unit 401 determines whether or not the value of SCAN_NO is 1 (step S508a). If the value of SCAN_NO is 1, the process proceeds to step S508c. If the value of SCAN_NO is not 1, the control unit 401 determines whether or not a reception level (received signal strength) of a frame of the search request response packet is equal to or greater than a predetermined level (step S508b). If the frame reception level is equal to or greater than the predetermined level, the process proceeds to step S508c. If the frame reception level is less than the predetermined level, the process returns to step S505. If the process proceeds to step S508c, the control unit 401 adds 1 to the value of TERM_NUM[SW_NO], and updates the value (step S508c). After the process of step S508c, the process returns to step S509.

The value of TERM_NUM[SW_NO] indicates the number of peripheral wireless communication terminals using the same communication channel (any one of the communication channels 1, 6, and 11) as the communication channel set by the CH setting switch or using a communication channel having a use frequency band overlapping that of the communication channel set by the CH setting switch. If the value of SCAN_NO is 1 in step S508a, another wireless communication terminal uses the same communication channel as the communication channel set by the CH setting switch. If the frame reception level is equal to or greater than the predetermined level in step S508b, another wireless communication terminal uses a communication channel having a use frequency band overlapping that of the communication channel set by the CH setting switch. Although a common threshold is set as a threshold of the frame reception level in each adjacent CH in this embodiment, a predetermined reception level threshold may be set for each adjacent CH.

If the process has proceeded to step S509, the control unit 401 according to the invention determines whether or not a predetermined time has elapsed after the transmission of the search request packet step S504 (step S509). If the predetermined time has not elapsed, the process returns to step 505. If the predetermined time has elapsed, the control unit 401 adds 1 to the value of SCAN_NO, and updates the value (step S510).

Subsequently, the control unit 401 reads a number of a communication channel (a communication channel for which the CH switch number is SW_NO and the search order is SCAN_NO) designated by SW_NO and SCAN_NO after update from the search table, and determines whether or not the number is 0 (step S511). If the number of the communication channel designated by SW_NO and SCAN_NO after the update is not 0, the process returns to step S503. If the number of the communication channel designated by SW_NO and SCAN_NO after the update is 0, the control unit 401 determines whether or not the communication channel set by the CH setting switch is available (step S512).

In this embodiment, if the number of wireless terminals, TERM_NUM[SW_NO], counted in step S508 is less than a predetermined threshold, the control unit 401 determines that the communication channel set by the CH setting switch is available. If it is determined that the communication channel set by the CH setting switch is available, the control unit 401 displays a use state of the communication channel set by the CH setting switch on the monitor 407 (step S513). At this time, the number of wireless communication terminals using each communication channel included in a communication channel group may be displayed by a numeric value, and a congestion degree of the communication channel may be graphically displayed on the basis of a preset threshold.

Subsequently, the control unit 401 reads a number of a communication channel (any one of the communication channels 1, 6, and 11) set by the CH setting switch from the communication channel setting table, reads communication setting parameters corresponding to the communication channel from the ROM 402, and sets the communication setting parameters to the wireless communication circuit unit 404 (step S514). Subsequently, the receiving device 200 makes the transition to a logical connection phase and executes a process of establishing a logical connection (step S515).

In the logical connection, a state is reached in which a combination of two specific wireless communication terminals, that is, the endoscope 100 and the receiving device 200, among a plurality of communication terminals physically connected is fixed. In this embodiment, if the logical connection is completed, a destination (medium access control (MAC) address) to which the endoscope 100 transmits image data is fixed. In the logical connection phase, the endoscope 100 transmits a MAC address request packet including a MAC address of the endoscope 100, and the receiving device 200 receiving the MAC address request packet transmits a MAC address request response packet including a MAC address of the receiving device 200, so that the MAC addresses are exchanged between the endoscope 100 and the receiving device 200.

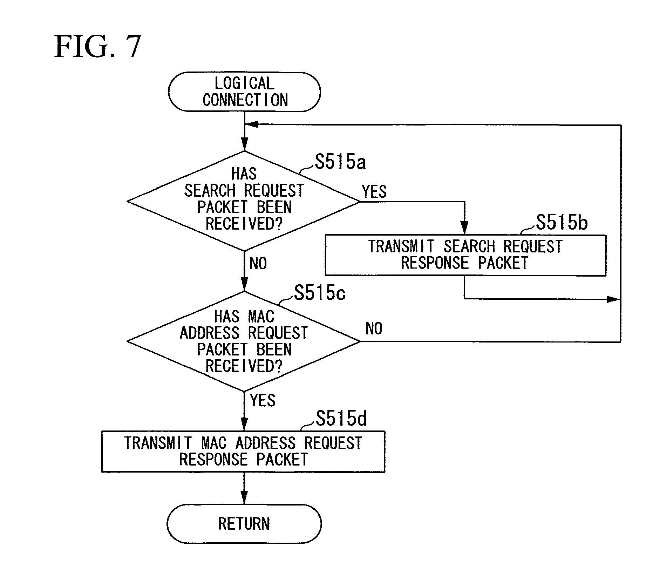

FIG. 7 shows details of step S515 shown in FIG. 5. In the logical connection, first, the control unit 401 determines whether or not the search request packet has been received from another wireless communication terminal after communication channel setup (step S515a). If the search request packet has been received, the control unit 401 causes the wireless communication circuit unit 404 to transmit a search request response packet in a unicast scheme (S515b). Thereafter, the process returns to step S515a. If the search request packet has not been received, the control unit 401 determines whether or not a MAC address request packet has been received from another wireless communication terminal (step S515c). If the MAC address request packet has been received, the control unit 401 causes the wireless communication circuit unit 404 to transmit a MAC address request response packet in a unicast scheme (step S515d). On the other hand, if the MAC address request packet has not been received, the process returns to step S515a.

Returning to FIG. 5, the wireless communication circuit unit 404 starts to receive image data transmitted from the endoscope 100 if the logical connection is completed (step S516).

If it is determined that the communication channel set by the CH setting switch is unavailable in step S512, the control unit 401 reports that a communication environment of the communication channel set by the CH setting switch is bad and unavailable, and causes the monitor 407 to display an alarm message for an instruction to change the CH setting switch (step S517). For example, a message "The set communication channel is unavailable. Please change the CH setting switch," is displayed on the monitor 407.

Subsequently, the control unit 401 determines whether or not a state of the CH setting switch has been changed (step S518). If the state of the CH setting switch has not been changed, the process of step S517 is re-executed. If the state of the CH setting switch has been changed, the process returns to step S502.

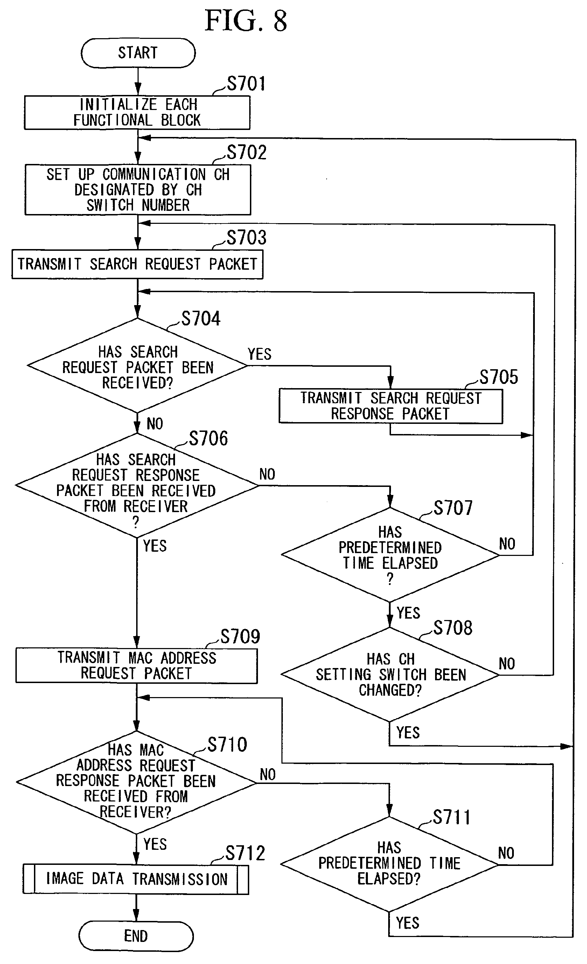

Hereinafter, an operation of the endoscope 100 will be described according to FIG. 8. The operator turns on power of the endoscope 100 after setting a communication channel by the CH setting switch 103 of the endoscope 100 according to a communication channel displayed on the communication setting display unit 201 of the receiving device 200. If the endoscope 100 is powered on, the control unit 301 initializes each functional block of the endoscope 100 (step S701).

Subsequently, the endoscope 100 makes the transition to the search phase of the wireless communication terminal. Upon transition to the search phase, the control unit 301 reads a number of a communication channel (any one of the communication channels 1, 6, and 11) set by the CH setting switch 103 from the search table, reads communication setting parameters corresponding to the communication channel from the ROM 302, and sets the communication setting parameters to the wireless communication circuit unit 306 (step S702). In a search for a wireless communication terminal, a search request packet is transmitted and a search request response packet to the search request packet is received during a predetermined period. Thus, the control unit 401 causes the wireless communication circuit unit 404 to transmit the search request packet in a broadcast scheme (step S703).

After the transmission of the search request packet, the control unit 301 determines whether or not a search request packet from another wireless communication terminal has been received (step S704). If the search request packet has been received, the control unit 301 causes the wireless communication circuit unit 306 to transmit a search request response packet in a unicast scheme (step S705). Thereafter, the process returns to step S704. If the packet request packet has not been received, the control unit 301 determines whether or not a search request response packet from a receiver has been received (step S706).

If the search request response packet from the receiver has been received, the endoscope 100 makes the transition to a logical connection phase, and executes a process of establishing the logical connection to be described below (steps S709 to S711). That is, the control unit 401 causes the wireless communication circuit unit 404 to transmit a MAC address request packet in a broadcast scheme (step S709). After the transmission of the MAC address request packet, the control unit 301 determines whether or not a MAC address request response packet has been received (step S710). If the MAC address request response packet from the receiver has been received, that is, if the logical connection is completed, the wireless communication circuit unit 306 starts to transmit image data to the receiving device 200 (step S712).

If the search request response packet has not been received, the control unit 401 determines whether or not a predetermined time has elapsed after the transmission of the search request packet in step S703 (step S707). If the predetermined time has not elapsed, the process returns to step S704. If the predetermined time has elapsed, the control unit 401 determines whether or not a state of the CH setting switch 103 has been changed (step S708). If the state of the CH setting switch 103 has not been changed, the process returns to step S703. If the state of the CH setting switch 103 has been changed, the process returns to step S702.

Likewise, if the MAC address request response packet has not been received, the control unit 401 determines whether or not a predetermined time has elapsed after the transmission of the MAC address request response packet of step S709 (step S711). If the predetermined time has not elapsed, the process returns to step S710. If the predetermined time has elapsed, the process returns to step S702.

In the first operation example, the control unit 401 of the receiving device 200 selects a communication channel group designated by the CH setting switch (step S503). The control unit 401 detects a use state of a communication channel belonging to the communication channel group (the number of peripheral wireless communication terminals using the same communication channel (any one of the communication channels 1, 6, and 11) as the communication channel set by the CH setting switch or using a communication channel having a use frequency band overlapping that of the communication channel set by the CH setting switch) (steps S504 to S511). Furthermore, the control unit 401 determines whether or not to perform communication using the communication channel belonging to the selected communication channel group on the basis of a detection result of the use state of the communication channel (step S512).

As described above, it is possible to efficiently select a communication channel having a good communication state by detecting the use state of only the communication channel belonging to the communication channel group. Furthermore, it is possible to shorten a connection waiting time of the endoscope 100 driven by a battery and implement power saving by turning on the power of the endoscope 100 after a communication channel to be used by the receiving device 200 is fixed. It is possible to reduce the occurrence of a communication error because a communication channel in which radio wave interference is not easily generated for communication channels used by peripheral wireless communication terminals is selected in the determination of step S512.

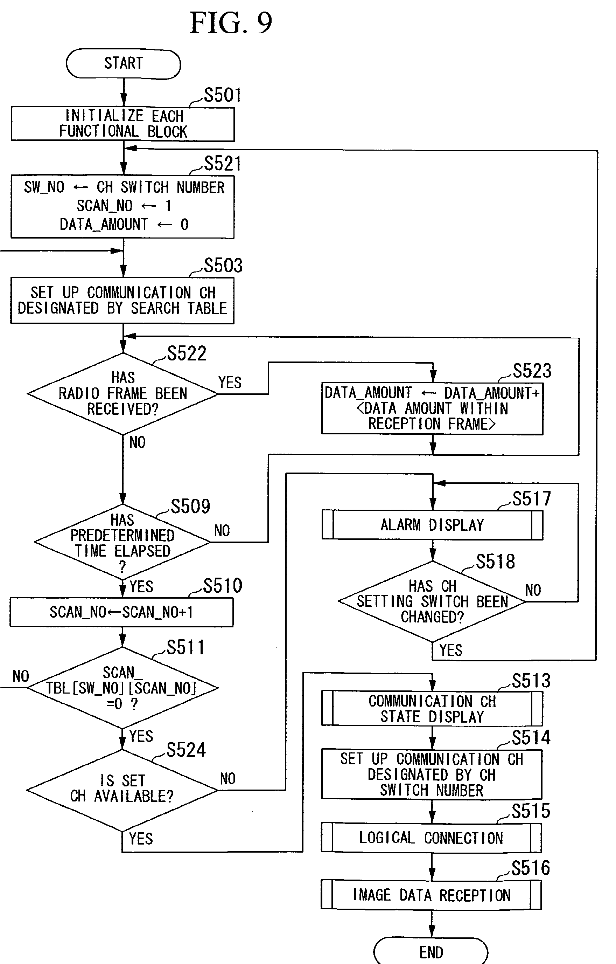

Next, a second operation example will be described. Because an operation of the endoscope 100 in the second operation example is the same as in the first operation example, a description thereof is omitted. Hereinafter, an operation of the receiving device 200 will be described according to FIG. 9. In FIG. 9, the same processes as those shown in FIG. 5 are denoted by the same step numbers. Hereinafter, only steps different from those shown in FIG. 5 will be described.

After the process of step S501, the control unit 401 initializes parameters SW_NO, SCAN_NO, and DATA_AMOUNT to be later used in control (step S521). SW_NO is a parameter for storing a CH switch number, and is stored as a CH switch number set by the CH setting switch during initialization. SCAN_NO is a parameter for storing a search order in the search table, and is stored as 1 during the initialization. DATA_AMOUNT is a parameter for storing a data amount.

After the process of step S521, the process proceeds to step S503. After the process of step S503, the control unit 401 determines whether or not a radio frame from another wireless communication terminal has been received (step S522). If the radio frame from another wireless communication terminal has been received, the control unit 401 adds a data amount within the received frame to the value of DATA_AMOUNT and updates the value (step S523). After the process of step S523, the process returns to step S522. If the radio frame from another wireless communication terminal has not been received, the process proceeds to step S509.

If a number of a communication channel designated by SW_NO and SCAN_NO after the update is 0 in step S511, the control unit 401 determines whether or not the communication channel set by the CH setting switch is available (step S524). While the value of TERM_NUM[SW_NO] is used in the determination in step S512 of FIG. 5, the value of DATA_AMOUNT is used in the determination in step S524 of FIG. 9. The value of DATA_AMOUNT indicates an amount of data transmitted using the same communication channel (any one of the communication channels 1, 6, and 11) as the communication channel set by the CH setting switch or using a communication channel having a use frequency band overlapping that of the communication channel set by the CH setting switch.

If the value of DATA_AMOUNT counted in step S523 is less than a predetermined threshold, the control unit 401 determines that the communication channel set by the CH setting switch is available. If it is determined that the communication channel set by the CH setting switch is available, the process proceeds to step S513. If it is determined that the communication channel set by the CH setting switch is unavailable, the process proceeds to step S517.

In the second operation example, the control unit 401 of the receiving device 200 selects a communication channel group designated by the CH setting switch (step S503). The control unit 401 detects a use state of a communication channel belonging to the communication channel group (an amount of data transmitted using the same communication channel as the communication channel set by the CH setting switch or using a communication channel having a use frequency band overlapping that of the communication channel set by the CH setting switch) (steps S522, S523, and S509 to S511). Furthermore, the control unit 401 determines whether or not to perform communication using the communication channel belonging to the communication channel group (step S524).

Therefore, in the second operation example, it is also possible to efficiently select a communication channel having a good communication state and implement power saving of the endoscope 100. Also, it is possible to reduce the occurrence of a communication error.

Next, a third operation example will be described. In the third operation example, an automatic mode in which the receiving device 200 can automatically select a communication channel is provided in addition to a mode in which the operator can manually select the CHs 1 to 3 by the CH setting switch. Thus, a switch by which the automatic mode is selectable is added to the operation unit 408 of the receiving device 200.

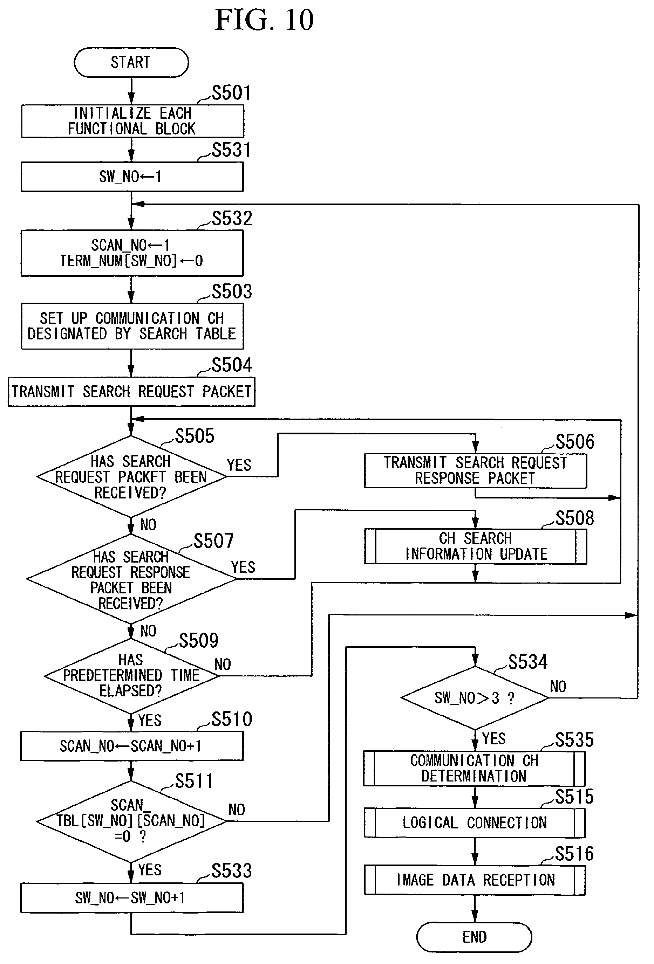

Because the operation of the endoscope 100 in the third operation example is the same as in the first operation example, description thereof is omitted. Hereinafter, an operation of the receiving device 200 in the automatic mode will be described according to FIG. 10. In FIG. 10, the same processes as those shown in FIG. 5 are denoted by the same step numbers. Hereinafter, only steps different from those shown in FIG. 5 will be described.

After the process of step S501, the control unit 401 initializes a parameter SW_NO to be later used in control (step S531), and further initializes parameters SCAN_NO and TERM_NUM[SW_NO] (step S532). SW_NO is a parameter for storing a CH switch number, and is stored as 1 during initialization. SCAN_NO is a parameter for storing a search order in the search table, and is stored as 1 during the initialization. TERM_NUM[SW_NO] is a parameter for storing the number of peripheral wireless communication terminals using a communication channel satisfying a predetermined condition, and is stored as 0 during the initialization. TERM_NUM[SW_NO] includes three values of TERM_NUM[1], TERM_NUM[2], and TERM_NUM[3].

In the first operation example, at this time, an LED corresponding to the set communication channel is turned on in the communication setting display unit 201 to indicate communication setting set by the CH setting switch. However, at this time in the third operation example, the LED of the communication setting display unit 201 is in a turn-off state because a communication channel to be used is not fixed.

If a number of a communication channel designated by SW_NO and SCAN_NO after update is 0 in step S511, the control unit 401 adds 1 to the value of SW_NO and updates the value (step S533). Subsequently, the control unit 401 determines whether or not the value of SW_NO exceeds 3 (step S534).

If the value of SW_NO is equal to or less than 3, the process returns to step S532. If the value of SW_NO exceeds 3, the control unit 401 determines a communication channel to be used on the basis of the value of TERM_NUM[SW_NO]. Specifically, the control unit 401 compares the number of wireless communication terminals, TERM_NUM[1], TERM_NUM[2], and TERM_NUM[3], counted in step S508 for each selectable communication channel, and determines a communication channel corresponding to the smallest number of wireless communication terminals as the communication channel to be used (step S535).

Specifically, the communication channel 1 corresponding to the CH switch number 1 is selected if TERM_NUM[1] is smallest, the communication channel 6 corresponding to the CH switch number 2 is selected if TERM_NUM[2] is smallest, and the communication channel 11 corresponding to the CH switch number 3 is selected if TERM_NUM[3] is smallest. After the process of step S535, the process proceeds to step S515. Because the process of step S515 is the same as described above with reference to FIG. 7, description thereof is omitted. At this time, communication setting parameters of the determined communication channel are set in the wireless communication circuit unit 404, and an LED corresponding to the set communication channel is turned on in the communication setting display unit 201.

Therefore, in the third operation example, it is also possible to efficiently select a communication channel having a good communication state and implement power saving of the endoscope 100. Also, it is possible to reduce the occurrence of a communication error.

Next, a fourth operation example will be described. In the fourth operation example, like the third operation example, an automatic mode in which the receiving device 200 can automatically select a communication channel is provided in addition to a mode in which the operator can manually select the CHs 1 to 3 by the CH setting switch. In the fourth operation example, a method shown in the second operation example is used as a method of detecting a use state of the communication channel.

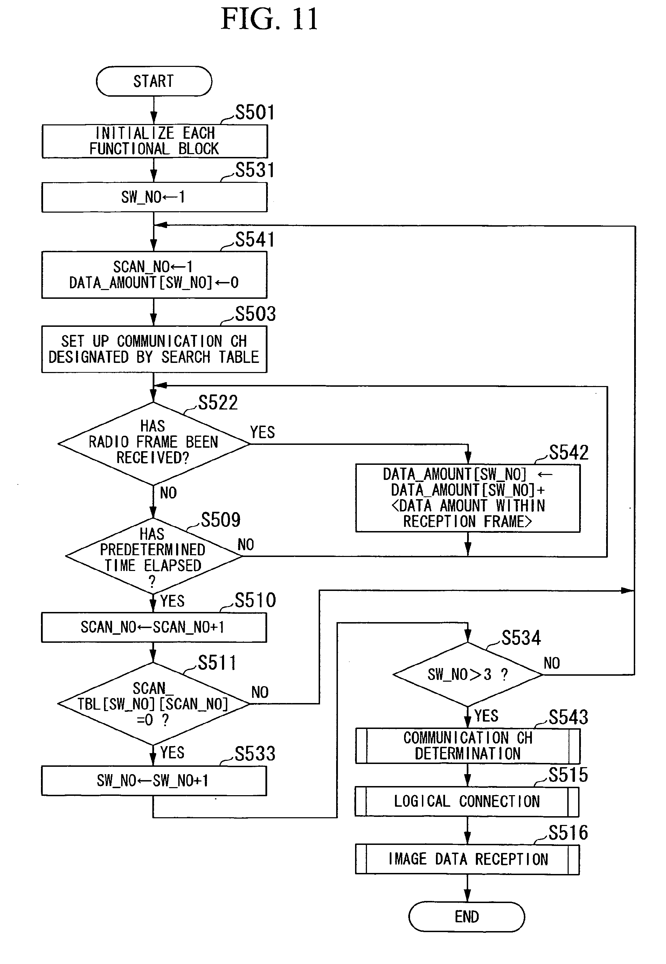

Because the operation of the endoscope 100 in the fourth operation example is the same as in the first operation example, description thereof is omitted. Hereinafter, an operation of the receiving device 200 in the automatic mode will be described according to FIG. 11. In FIG. 11, the same processes as those shown in FIGS. 9 and 10 are denoted by the same step numbers. Hereinafter, only steps different from those shown in FIGS. 9 and 10 will be described.

After the process of step S531, the control unit 401 initializes parameters SCAN_NO and DATA_AMOUNT[SW_NO] to be later used in control (step S541). SCAN_NO is a parameter for storing a search order in the search table, and is stored as 1 during initialization. DATA_AMOUNT[SW_NO] is a parameter for storing a data amount, and is stored as 0 during the initialization. DATA_AMOUNT[SW_NO] includes three values of DATA_AMOUNT[1], DATA_AMOUNT[2], and DATA_AMOUNT[3].

If a radio frame from another wireless communication terminal has been received in step S522, the control unit 401 adds a data amount within the received frame to the value of DATA_AMOUNT[SW_NO], and updates the value (step S542). After the process of step S542, the process returns to step S522. If the radio frame from another wireless communication terminal has not been received, the process proceeds to step S509.

If the value of SW_NO exceeds 3 in step S534, the control unit 401 determines a communication channel to be used on the basis of the value of DATA_AMOUNT[SW_NO]. Specifically, the control unit 401 compares DATA_AMOUNT[1], DATA_AMOUNT[2], and DATA_AMOUNT[3] counted in step S542 for each selectable communication channel, and determines a communication channel corresponding to a smallest value as the communication channel to be used (step S543).

Specifically, the communication channel 1 corresponding to the CH switch number 1 is selected if DATA_AMOUNT[1] is smallest, the communication channel 6 corresponding to the CH switch number 2 is selected if DATA_AMOUNT[2] is smallest, and the communication channel 11 corresponding to the CH switch number 3 is selected if DATA_AMOUNT[3] is smallest. After the process of step S543, the process proceeds to step S515. Because the process of step S515 is the same as described above with reference to FIG. 7, description thereof is omitted. At this time, communication setting parameters of the determined communication channel are set in the wireless communication circuit unit 404, and an LED corresponding to the set communication channel is turned on in the communication setting display unit 201.

In the fourth operation example, it is also possible to efficiently select a communication channel having a good communication state and implement power saving of the endoscope 100. Also, it is possible to reduce the occurrence of a communication error.

In the above-described four operation examples, a communication channel set by the CH setting switch and all communication channels each having a use frequency band partially overlapping that of the set communication channel are objects of which communication states are to be sensed as a communication channel group. On the other hand, as shown in FIG. 18, it is possible to further shorten a time in which it is determined whether or not a communication channel is available by excluding a communication channel having a small overlap range in a frequency band and small radio wave interference to the communication channel (for example, the communication channel 1) set by the CH setting switch from the objects of which communication states are to be sensed.

In this case, the communication channel setting table becomes as shown in FIG. 14, and the search table becomes as shown in FIG. 15. Although FIGS. 12 and 13 include communication channels (the communication channels 3, 4, 8, and 9) belonging to two different communication channel groups, all communication channels in FIGS. 14 and 15 belong to only one communication channel group.

According to this embodiment as described above, a use state of a communication channel belonging to a selected communication channel group is detected, and it is determined whether or not to perform communication using the communication channel belonging to the selected communication channel group on the basis of a detection result. Thereby, it is possible to efficiently select a communication channel having a good communication state as compared to the case where all communication channels are searched for. Furthermore, it is possible to shorten a connection waiting time of the endoscope 100 driven by the battery and implement power saving by turning on the power of the endoscope 100 after a communication channel to be used by the receiving device 200 is fixed. Furthermore, it is possible to reduce the occurrence of a communication error because a communication channel in which radio wave interference is not easily generated for communication channels used by peripheral wireless communication terminals is selected.

If the communication channel set by the CH setting switch is unavailable, a message for an instruction of a change of the CH setting switch is displayed on the monitor 407 to prompt selection of a communication channel group different from the selected communication channel group (step S517). Thereby, the operator can easily know when to change a communication channel to be used in the logical connection.

If the communication channel set by the CH setting switch is available and the communication using the communication channel is determined to be performed, information regarding a use state of the communication channel to be used is displayed on the monitor 407 (step S513). Thereby, the operator can know the use state of the communication channel to be used. In the operations shown in FIGS. 5 and 9, a determination of a state of the CH setting switch as in step S518 is made after step S513. If the state of the CH setting switch has been changed, the process from step S502 may be re-executed. Thereby, if there is a communication channel having a better communication state than a communication channel determined to be available, it is possible to select the communication channel having the better communication state.

To detect the use state of the communication channel, a reception level (received signal strength) or communication traffic (data amount) of a communication channel belonging to a selected communication channel group is detected (steps S508, S523, and S542). Thereby, it is possible to easily detect the use state of the communication channel.

In the first operation example, TERM_NUM[SW_NO]=0 (SW_NO=1, 2, or 3) if there is no other wireless communication terminal using the same communication channel (any one of the communication channels 1, 6, and 11) as the communication channel set by the CH setting switch and there is no other wireless communication terminal using a communication channel having a use frequency band overlapping that of the communication channel set by the CH setting switch. At this time, it is possible to select a communication channel having a good communication state by use of the communication channel set by the CH setting switch.

In the second operation example, it is possible to select a communication channel having a good communication state by use of the communication channel set by the CH setting switch when detected communication traffic (data amount) is less than a predetermined value.

It is possible to select a communication channel having the best communication state by use of a communication channel corresponding to a smallest value of TERM_NUM[SW_NO] (SW_NO=1, 2, 3), which is the number of peripheral wireless communication terminals using a communication channel satisfying a predetermined condition.

In the fourth operation example, it is possible to select a communication channel having the best communication state by use of a communication channel corresponding to a smallest value of a data amount DATA_AMOUNT [SW_NO] (SW_NO=1, 2, 3) of a specific communication channel.

In the communication channel setting table shown in FIG. 14, as compared to the communication channel setting table shown in FIG. 12, communication channels (for example, the communication channels 3 and 9) each having an overlap of a use frequency band with that of the communication channel (for example, the communication channel 6) set by the CH setting switch that is equal to or less than a predetermined range are excluded. As a result, each of individual communication channels belonging to each communication channel group (for example, the communication channels 2 and 3 corresponding to the CH switch number 1) is set to have a use frequency band, which does not overlap those of communication channels belonging to other communication channel groups (for example, the communication channel 6 corresponding to the CH switch number 2 and the communication channel 11 corresponding to the CH switch number 3) set by the CH setting switch. Thereby, because a communication channel having a small influence on radio wave interference to the communication channel set by the CH setting switch is excluded from each communication channel group in FIG. 14, the number of communication channels belonging to an individual communication channel group is less than in FIG. 12. Therefore, it is possible to efficiently select a communication channel in which occurrence radio wave interference is difficult.

As shown in FIGS. 12 and 13, communication channels (for example, the communication channels 3, 4, 5, 7, 8, and 9) each having an overlap of a use frequency band with that of a communication channel (for example, the communication channel 6) set by the CH setting switch that is equal to or greater than a predetermined range (about 5 MHz or more in this embodiment) are set to belong to the same communication channel group so that it is possible to efficiently detect a use state of a communication channel in which radio wave interference with a communication channel (for example, the communication channel 6) to be used occurs. Therefore, it is possible to efficiently select a communication channel having a good communication state.

Although the embodiments of the present invention, which is defined by the claims, have been described above in detail with reference to the drawings, specific configurations are not limited to those of the embodiments.

### INDUSTRIAL APPLICABILITY

The present invention is applicable in wireless communication based on a wireless communication system such as a wireless LAN.

### DESCRIPTION OF THE REFERENCE SYMBOLS

100 : Endoscope (Transmitting device)
101 : Power switch
102 : Operating switch
103 : CH setting switch
104 : State indication LED
200 : Receiving device
201 : Communication setting display unit
301, 401 : Control unit (Determination unit)
302, 402 : ROM (Storage unit)
303,403 : RAM
304 : Image pickup unit
305 : Illumination unit
306, 404 : Wireless communication circuit unit (Communication unit)
307,405 : Antenna
308, 408 : Operation unit
406 : Image signal processing unit
407 Monitor (Output unit)

## Claims

1. A receiving device (200) of a wireless endoscopic apparatus configured to use a plurality of communication channels, each of which has a use frequency band partially overlapping with that of at least one of the other communication channels, wherein one communication channel group among the plurality of communication channels includes a first communication channel used for logical connection with a transmitting device (100) and an adjacent communication channel having a use frequency band partially overlapping with that of the first communication channel, the receiving device (200) comprising:
a communication unit (404) configured to communicate with the transmitting device (100) by using the first communication channel, the communication unit (404) being configured to receive image data transmitted from the transmitting device (100);
a storage unit (402) configured to store a plurality of communication channel groups;
an operation unit (408) comprising a channel setting switch (103) configured to:
- receive an operator selection of a channel switch number corresponding to an arbitrary communication channel group from among the plurality of communication channel groups stored in the storage unit (402), and
- transfer the selection result to a control unit (401); and
the control unit (401) configured to perform a detection of use states of the first communication channel and the adjacent communication channels belonging to the communication channel group that has been selected, the control unit (401) being further configured to determine whether or not to perform a communication using the first communication channel belonging to the communication channel group that has been selected based on results of the detection, wherein the control unit (401), when to determine whether or not to perform a communication using the first communication channel, is further configured to:
- transmit, by the communication unit (404), a search request packet;
- determine whether or not a search request response packet from another wireless communication terminal has been received;
- execute, if the search request response packet has been received, a process of updating a number of peripheral wireless communication terminals using the same communication channel by:
-- determining whether or not the value of a first parameter for storing a search order (SCAN_NO) in a search table is a predetermined value, wherein the predetermined value being 1 corresponds to the first communication channel of the communication channel group set by the channel setting switch (103);
-- determining, if the value of the first parameter is not the predetermined value, whether or not a reception level of a frame of the search request response packet is equal to or greater than a predetermined level;
-- incrementing, if the frame reception level is equal to or greater than the predetermined level or if the value of the first parameter is the predetermined value, the number of peripheral wireless communication terminals using the same communication channel by 1; and
-- returning, after the incrementing operation or if the frame reception level is not equal to or greater than the predetermined level, to the operation of determining whether or not a search request packet from another wireless endoscopic device has been received; and
- determine, after a predetermined time has elapsed, if the number of peripheral wireless communication terminals using the same communication channel is less than a predetermined threshold, that the first communication channel is available.

2. The receiving device (200) of the wireless endoscopic apparatus according to claim 1, wherein
the storage unit (402) is configured to store the first communication channel having a use frequency band that is not overlapping with that of another first communication channel included in another communication channel group.

3. The receiving device (200) of the wireless endoscopic apparatus according to claim 1, further comprising:
an output unit (407) configured to output information for prompting selection of a communication channel group that is different from the communication channel group that has been selected by the operation unit (408) if the control unit (401) determines not to perform the communication.

4. The receiving device (200) of the wireless endoscopic apparatus according to claim 3, wherein if the control unit (401) determines to perform the communication, then the output unit (407) is configured to output information regarding a use state of a communication channel to perform the communication.

5. The receiving device (200) of the wireless endoscopic apparatus according to claim 1, wherein the control unit (401) is configured to detect communication traffic of a communication channel belonging to the communication channel group that has been selected as the use states.

6. The receiving device (200) of the wireless endoscopic apparatus according to claim 1, wherein the control unit (401) is configured to detect received signal strength of a communication channel belonging to the communication channel group that has been selected as the use states.

7. The receiving device (200) of the wireless endoscopic apparatus according to claim 5, wherein the control unit (401) is configured to determine whether or not to perform communication using a first communication channel belonging to the communication channel group that has been selected based on whether or not the communication traffic is less than a predetermined threshold.

8. The receiving device (200) of the wireless endoscopic apparatus according to claim 6, wherein the control unit (401) is configured to determine whether or not to perform communication using a first communication channel belonging to the communication channel group that has been selected based on whether or not the received signal strength is less than a predetermined threshold.

9. The receiving device (200) of the wireless endoscopic apparatus according to claim 1, wherein the storage unit (402) is configured to store a first communication channel and an adjacent communication channel that are belonging to the same communication channel group, wherein the overlap is equal to or greater than a predetermined range.

10. The receiving device (200) of the wireless endoscopic apparatus according to claim 9, wherein the storage unit (402) is configured to store a first communication channel and an adjacent communication channel that are belonging to different communication channel groups, the adjacent communication channel having a use frequency band not overlapping with that of the first communication channel.

11. A receiving method for use in a receiving device (200) of a wireless endoscopic apparatus, the method comprising the steps of:
using a plurality of communication channels, each of which has a use frequency band partially overlapping with that of at least one of the other communication channels, wherein one communication channel group among the plurality of communication channels includes a first communication channel used for logical connection with a transmitting device (100) and an adjacent communication channel having a use frequency band partially overlapping with that of the first communication channel;
communicating with the transmitting device (100) by using the first communication channel;
receiving image data transmitted from the transmitting device (100);
storing a plurality of communication channel groups;
receiving an operator selection of a channel switch number corresponding to an arbitrary communication channel group from among the plurality of communication channel groups that have been stored;
transfering the selection result to a control unit;
performing, by the control unit, a detection of use states of the first communication channel and the adjacent communication channels belonging to the communication channel group that has been selected;
determining, by the control unit, whether or not to perform a communication using the first communication channel belonging to the communication channel group that has been selected based on results of the detection, the determining whether or not to perform a communication using the first communication channel comprises:
transmitting (S504), by the communication unit (404), a search request packet;
determining (S507) whether or not a search request response packet from another wireless communication terminal has been received;
executing (S508), if the search request response packet has been received, a process of updating a number of peripheral wireless communication terminals using the same communication channel by:
- determining (S508a) whether or not the value of a first parameter for storing a search order (SCAN_NO) in a search table is a predetermined value, wherein the predetermined value being 1 corresponds to the first communication channel of the communication channel group set by the channel setting switch (103);
- determining (S508b), if the value of the first parameter is not the predetermined value, whether or not a reception level of a frame of the search request response packet is equal to or greater than a predetermined level;
-- incrementing (S508c), if the frame reception level is equal to or greater than the predetermined level or if the value of the first parameter is the predetermined value, the number of peripheral wireless communication terminals using the same communication channel by 1; and
-- returning (S508), after the incrementing operation or if the frame reception level is not equal to or greater than the predetermined level, to the operation of determining whether or not a search request packet from another wireless endoscopic device has been received; and
- determining (S509), after a predetermined time has elapsed, if the number of peripheral wireless communication terminals using the same communication channel is less than a predetermined threshold, that the first communication channel is available.

12. A wireless endoscopic apparatus comprising the receiving device (200) of any of claims 1 to 10 and a transmitting device (100), wherein
the transmitting device (100) comprises
a second communication unit (306) configured to communicate with the receiving device by (200) use of the first communication channel and transmit the image data to the receiving device (200).

13. A computer program comprising instructions to carry out the steps of claim 11 when executed by the control unit (401) of the receiving device (200) of a wireless endoscopic apparatus according to any one of claims 1-10.

## Patentansprüche

1. Empfangsvorrichtung (200) einer drahtlosen endoskopischen Vorrichtung, die konfiguriert ist, um eine Vielzahl von Kommunikationskanälen zu verwenden, von denen jeder ein Verwendungsfrequenzband umfasst, das teilweise mit jenem von zumindest einem weiteren der anderen Kommunikationskanäle überlappt, wobei eine einzelne Kommunikationskanalgruppe unter der Vielzahl von Kommunikationskanälen einen ersten Kommunikationskanal, der für eine logische Verbindung mit einer Sendevorrichtung (100) verwendet wird, und einen angrenzenden Kommunikationskanal umfasst, der ein Verwendungsfrequenzband aufweist, das teilweise mit jenem des ersten Kommunikationskanals überlappt, wobei die Empfangsvorrichtung (200) umfasst:
eine Kommunikationseinheit (404), die konfiguriert ist, mit der Sendevorrichtung (100) unter Verwendung des ersten Kommunikationskanals zu kommunizieren, wobei die Kommunikationseinheit (404) konfiguriert ist, um Bilddaten zu empfangen, die von der Sendevorrichtung (100) gesendet sind;
eine Speichereinheit (402), die konfiguriert ist, um eine Vielzahl von Kommunikationskanalgruppen zu speichern;
eine Bedieneinheit (408), die einen Kanalsetzschalter (103) umfasst, und die konfiguriert ist, um:
- eine Bedienerauswahl einer Kanalschalterzahl entsprechend einer beliebigen Kommunikationskanalgruppe aus der Vielzahl von Kommunikationskanalgruppen zu empfangen, die in der Speichereinheit (402) gespeichert sind, und
- das Auswahlergebnis zu einer Steuereinheit (401) zu übermitteln; und
wobei die Steuereinheit (401) konfiguriert ist, um eine Erfassung von Verwendungszuständen des ersten Kommunikationskanals und der angrenzenden Kommunikationskanäle durchzuführen, die zu der Kommunikationskanalgruppe gehören, die ausgewählt wurde, wobei die Steuereinheit (401) weiterhin konfiguriert ist, um zu bestimmen, ob eine Kommunikation unter Verwendung des ersten Kommunikationskanals, der zu der Kommunikationskanalgruppe gehört, die auf der Grundlage der Erfassung ausgewählt wurde, durchzuführen ist oder nicht, wobei die Steuereinheit (401), wenn bestimmt wird, ob eine Kommunikation unter Verwendung des ersten Kommunikationskanals durchgeführt wird oder nicht, weiterhin konfiguriert ist, um:
- durch die Kommunikationseinheit (404) ein Suchanforderungspaket zu senden;
- zu bestimmen, ob ein Suchanforderungsantwortpaket von einem weiteren drahtlosen Kommunikationsendgerät empfangen wurde oder nicht;
- falls das Suchanforderungsantwortpaket empfangen wurde, einen Prozess zum Aktualisieren einer Anzahl von peripheren drahtlosen Kommunikationsendgeräten, die denselben Kommunikationskanal verwenden, auszuführen durch:
-- Bestimmen, ob der Wert eines ersten Parameters zum Speichern einer Suchreihenfolge (SCAN_MO) in einer Suchtabelle ein vorbestimmter Wert ist oder nicht, wobei der vorbestimmte Wert von 1 dem ersten Kommunikationskanal der Kommunikationskanalgruppe entspricht, die durch den Kanalsetzschalter (103) gesetzt ist;
-- Bestimmen, falls der Wert des ersten Parameters nicht der vorbestimmte Wert ist, ob ein Empfangspegel eines Rahmens des Suchanforderungsantwortpakets größer oder gleich einem vorbestimmten Pegel ist oder nicht;
-- Inkrementieren, falls der Rahmenempfangspegel größer oder gleich dem vorbestimmten Pegel ist oder falls der Wert des ersten Parameters der vorbestimmte Wert ist, der Anzahl peripherer drahtloser Kommunikationsendgeräte, die denselben Kommunikationskanal verwenden, um 1; und
-- Zurückkehren, nach dem Inkrementierungsvorgang oder falls der Rahmenempfangspegel nicht größer oder gleich dem vorbestimmten Pegel ist, zu dem Vorgang des Bestimmens, ob ein Suchanforderungspaket von einer weiteren drahtlosen endoskopischen Vorrichtung empfangen wurde oder nicht; und
- nach Ablauf einer vorbestimmten Zeit zu bestimmen, falls die Anzahl peripher drahtloser Kommunikationsendgeräte, die denselben Kommunikationskanal verwenden, weniger als ein vorbestimmter Schwellwert beträgt, dass der erste Kommunikationskanal zur Verfügung steht.

2. Empfangsvorrichtung (200) der drahtlosen endoskopischen Vorrichtung gemäß Anspruch 1, wobei
die Speichereinheit (402) konfiguriert ist, um den ersten Kommunikationskanal, der ein Verwendungsfrequenzband aufweist, das nicht mit jenem eines weiteren ersten Kommunikationskanals überlappt, der in einer anderen Kommunikationskanalgruppe umfasst ist, zu speichern.

3. Empfangsvorrichtung (200) der drahtlosen endoskopischen Vorrichtung gemäß Anspruch 1, weiterhin umfassend:
eine Ausgabeeinheit (407), die konfiguriert ist, um Informationen zum Veranlassen einer Auswahl einer Kommunikationskanalgruppe auszugeben, die von der Kommunikationskanalgruppe verschieden ist, die durch die Bedieneinheit (408) ausgewählt wurde, falls die Steuereinheit (401) bestimmt, dass die Kommunikation nicht durchzuführen ist.

4. Empfangsvorrichtung (200) der drahtlosen endoskopischen Vorrichtung gemäß Anspruch 3, wobei, falls die Steuereinheit (401) bestimmt, dass die Kommunikation durchzuführen ist, dann die Ausgabeeinheit (407) konfiguriert ist, um Informationen hinsichtlich eines Verwendungszustands eines Kommunikationskanals zum Durchführen der Kommunikation auszugeben.

5. Empfangsvorrichtung (200) der drahtlosen endoskopischen Vorrichtung gemäß Anspruch 1, wobei die Steuereinheit (401) konfiguriert ist, um Kommunikationsverkehr eines Kommunikationskanals zu erfassen, der zu der Kommunikationskanalgruppe gehört, die als die Verwendungszustände ausgewählt wurde.

6. Empfangsvorrichtung (200) der drahtlosen endoskopischen Vorrichtung gemäß Anspruch 1, wobei die Steuereinheit (401) konfiguriert ist, um eine Empfangssignalstärke eines Kommunikationskanals zu erfassen, der zu der Kommunikationskanalgruppe gehört, die als die Verwendungszustände ausgewählt wurde.

7. Empfangsvorrichtung (200) der drahtlosen endoskopischen Vorrichtung gemäß Anspruch 5, wobei die Steuereinheit (401) konfiguriert ist, um zu bestimmen, ob eine Kommunikation unter Verwendung eines ersten Kommunikationskanals durchzuführen ist oder nicht, der zu der Kommunikationskanalgruppe gehört, die auf der Grundlage dessen ausgewählt wurde, ob der Kommunikationsverkehr weniger als einen vorbestimmten Schwellwert beträgt oder nicht.

8. Empfangsvorrichtung (200) der drahtlosen endoskopischen Vorrichtung gemäß Anspruch 6, wobei die Steuereinheit (401) konfiguriert ist, um zu bestimmen, ob eine Kommunikation unter Verwendung eines ersten Kommunikationskanals durchzuführen ist oder nicht, der zu der Kommunikationskanalgruppe gehört, die auf der Grundlage dessen ausgewählt wurde, ob die Empfangssignalstärke weniger als einen vorbestimmten Schwellwert beträgt oder nicht.

9. Empfangsvorrichtung (200) der drahtlosen endoskopischen Vorrichtung gemäß Anspruch 1, wobei die Speichereinheit (402) konfiguriert ist, um einen ersten Kommunikationskanal und einen angrenzenden Kommunikationskanal zu speichern, die zu derselben Kommunikationskanalgruppe gehören, wobei die Überlappung größer oder gleich einem vorbestimmten Bereich ist.

10. Empfangsvorrichtung (200) der drahtlosen Vorrichtung gemäß Anspruch 9, wobei die Speichereinheit (402) konfiguriert ist, um einen ersten Kommunikationskanal und einen angrenzenden Kommunikationskanal zu speichern, die zu verschiedenen Kommunikationskanalgruppen gehören, wobei der angrenzende Kommunikationskanal ein Verwendungsfrequenzband aufweist, das nicht mit jenem des ersten Kommunikationskanals überlappt.

11. Empfangsverfahren zur Verwendung in einer Empfangsvorrichtung (200) in einer drahtlosen endoskopischen Vorrichtung, wobei das Verfahren die Schritte umfasst:
Verwenden einer Vielzahl von Kommunikationskanälen, von denen jeder ein Verwendungsfrequenzband aufweist, das teilweise mit jenem von zumindest einem der anderen Kommunikationskanäle überlappt, wobei eine einzelne Kommunikationskanalgruppe unter der Vielzahl von Kommunikationskanälen einen ersten Kommunikationskanal, der für eine logische Verbindung mit einer Sendevorrichtung (100) verwendet wird, und einen angrenzenden Kommunikationskanal umfasst, der ein Verwendungsfrequenzband umfasst, das teilweise mit jenem der ersten Kommunikationskanals überlappt;
Kommunizieren mit der Sendevorrichtung (100) unter Verwendung des ersten Kommunikationskanals;
Empfangen von Bilddaten, die von der Sendevorrichtung (100) gesendet sind;
Speichern einer Vielzahl von Kommunikationskanalgruppen;
Empfangen einer Bedienerauswahl einer Kanalschaltzahl entsprechend einer beliebigen Kommunikationskanalgruppe aus der Vielzahl von Kommunikationskanalgruppen, die gespeichert wurden;
Übermitteln des Auswahlergebnisses zu einer Steuereinheit;
Durchführen, durch die Steuereinheit, einer Erfassung von Verwendungszuständen des ersten Kommunikationskanals und der angrenzenden Kommunikationskanäle, die zu der Kommunikationskanalgruppe gehören, die ausgewählt wurden;
Bestimmen, durch die Steuereinheit, ob eine Kommunikation unter Verwendung des ersten Kommunikationskanals durchzuführen ist oder nicht, der zu der Kommunikationskanalgruppe, die auf der Grundlage der Erfassung ausgewählt wurde, wobei das Bestimmen, ob eine Kommunikation unter Verwendung des ersten Kommunikationskanals durchzuführen ist oder nicht, umfasst:
Senden (S504), durch die Kommunikationseinheit (404), eines Suchanforderungspakets;
Bestimmen (S507), ob ein Suchanforderungsantwortpaket von einer weiteren drahtlosen Kommunikationsendgerät empfangen wurde oder nicht;
Ausführen (S508), falls das Suchanforderungsantwortpaket empfangen wurde, eines Prozesses zum Aktualisieren einer Anzahl von peripheren drahtlosen Kommunikationsendgeräten, die denselben Kommunikationskanal verwenden, durch:
- Bestimmen (S508a), ob der Wert eines ersten Parameters zum Speichern einer Suchreihenfolge (SCAN_MO) in einer Suchtabelle ein vorbestimmter Wert ist oder nicht, wobei der vorbestimmte Wert von 1 dem ersten Kommunikationskanal der Kommunikationskanalgruppe entspricht, die durch den Kanalsetzschalter (103) gesetzt ist;
- Bestimmen (S508d), falls der Wert des ersten Parameters nicht der vorbestimmte Wert ist, ob ein Empfangspegel eines Rahmens des Suchanforderungsantwortpakets größer oder gleich einem vorbestimmten Pegel ist oder nicht;
-- Inkrementieren (S508c), falls der Rahmenempfangspegel größer oder gleich dem vorbestimmten Pegel ist oder falls der Wert des ersten Parameters der vorbestimmte Wert ist, der Anzahl peripherer drahtloser Kommunikationsendgeräte, die denselben Kommunikationskanal verwenden, um 1; und
-- Zurückkehren (S508), nach dem Implementierungsvorgang oder falls der Rahmenempfangspegel nicht größer oder gleich dem vorbestimmten Pegel ist, zu dem Vorgang des Bestimmens, ob ein Suchanforderungspaket von einer weiteren drahtlosen endoskopischen Vorrichtung empfangen wurde oder nicht; und
- Bestimmen (S509), nach Ablauf einer vorbestimmten Zeit, falls die Anzahl peripherer drahtloser Kommunikationsendgeräte, die denselben Kommunikationskanal verwenden, weniger als ein vorbestimmter Schwellwert beträgt, dass der erste Kommunikationskanal zur Verfügung steht.

12. Drahtlose endoskopische Vorrichtung, umfassend die Empfangsvorrichtung (200) gemäß zumindest einem der Ansprüche 1 bis 10 und eine Sendevorrichtung (100), wobei
die Sendevorrichtung (100) umfasst:
eine zweite Kommunikationseinheit (306), die konfiguriert ist, um mit der Empfangsvorrichtung (200) durch Verwendung des ersten Kommunikationskanals zu kommunizieren und die Bilddaten zu der Empfangsvorrichtung (200) zu senden.

13. Computerprogram, das Anweisungen zum Ausführen der Schritte gemäß Anspruch 11 umfasst, bei Ausführung durch die Steuereinheit (401) der Empfangsvorrichtung (200) einer drahtlosen endoskopischen Vorrichtung gemäß zumindest einem der Ansprüche 1 bis 10.

## Revendications

1. Dispositif de réception (200) d'un appareil endoscopique sans fil configuré pour utiliser une pluralité de canaux de communication, dont chacun possède une bande de fréquence d'utilisation chevauchant partiellement celle d'au moins l'un parmi les autres canaux de communication, dans lequel un groupe de canaux de communication parmi la pluralité de canaux de communication comprend un premier canal de communication utilisé pour connexion logique avec un dispositif de transmission (100) et un canal de communication adjacent possédant une bande de fréquence d'utilisation chevauchant partiellement celle du premier canal de communication, le dispositif de réception (200) comprenant :
une unité de communication (404) configurée pour communiquer avec le dispositif de transmission (100) en utilisant le premier canal de communication, l'unité de communication (404) étant configurée pour recevoir des données d'image transmises depuis le dispositif de transmission (100) ;
une unité de mémoire (402) configurée pour mémoriser une pluralité de groupes de canaux de communication ;
une unité opérationnelle (408) comprenant un commutateur de réglage de canal (103) configuré pour :
- recevoir une sélection d'opérateur d'un numéro de commutateur de canal correspondant à un groupe de canaux de communication arbitraire parmi la pluralité de groupes de canaux de communication mémorisés dans l'unité de mémoire (402), et
- transférer le résultat de sélection vers une unité de commande (401) ; et
l'unité de commande (401) configurée pour réaliser une détection des états d'utilisation du premier canal de communication et des canaux de communication adjacents appartenant au groupe de canaux de communication qui a été sélectionné, l'unité de commande (401) étant en outre configurée pour déterminer s'il faut réaliser une communication en utilisant le premier canal de communication appartenant au groupe de canaux de communication qui a été sélectionné sur la base des résultats de la détection, dans lequel l'unité de commande (401), lorsqu'il est déterminé s'il faut réaliser une communication en utilisant le premier canal de communication, est en outre configurée pour :
- transmettre, par l'unité de communication (404), un paquet de demande de recherche ;
- déterminer si oui ou non un paquet de réponse à la demande de recherche provenant d'un autre terminal de communication sans fil a été reçu ;
- exécuter, si le paquet de réponse à la demande de recherche a été reçu, un processus de mise à jour d'un nombre de terminaux de communication sans fil périphériques utilisant le même canal de communication par les étapes consistant à :
-- déterminer si oui ou non la valeur d'un premier paramètre destiné à mémoriser un ordre de recherche (SCAN_NO) dans une table de recherche correspond à une valeur prédéterminée, dans lequel la valeur prédéterminée étant de 1 correspond au premier canal de communication du groupe de canaux de communication établi par le commutateur de réglage de canal (103) ;
-- déterminer, si la valeur du premier paramètre ne correspond pas à la valeur prédéterminée, si oui ou non un niveau de réception d'une trame du paquet de réponse à la demande de recherche est égal ou supérieur à un niveau prédéterminé ;
-- incrémenter, si le niveau de réception de trame est égal ou supérieur au niveau prédéterminé ou si la valeur du premier paramètre correspond à la valeur prédéterminée, le nombre de terminaux de communication sans fil périphériques utilisant le même canal de communication de 1 ; et
-- retourner, après l'opération d'incrémentation ou si le niveau de réception de trame n'est pas égal ou supérieur au niveau prédéterminé, à l'opération consistant à déterminer si oui ou non un paquet de demande de recherche provenant d'un autre dispositif endoscopique sans fil a été reçu ; et
- déterminer, après qu'un temps prédéterminé s'est écoulé, si le nombre de terminaux de communication sans fil périphériques utilisant le même canal de communication est inférieur à un seuil prédéterminé, que le premier canal de communication est disponible.

2. Dispositif de réception (200) de l'appareil endoscopique sans fil selon la revendication 1, dans lequel
l'unité de mémoire (402) est configurée pour mémoriser le premier canal de communication possédant une bande de fréquence d'utilisation qui ne chevauche pas celle d'un autre premier canal de communication contenu dans un autre groupe de canaux de communication.

3. Dispositif de réception (200) de l'appareil endoscopique sans fil selon la revendication 1, comprenant en outre :
une unité de sortie (407) configurée pour délivrer en sortie des informations pour inciter la sélection d'un groupe de canaux de communication qui est différent du groupe de canaux de communication qui a été sélectionné par l'unité opérationnelle (408) si l'unité de commande (401) détermine qu'il ne faut pas réaliser de communication.

4. Dispositif de réception (200) de l'appareil endoscopique sans fil selon la revendication 3, dans lequel si l'unité de commande (401) détermine qu'il faut réaliser la communication, alors l'unité de sortie (407) est configurée pour délivrer en sortie des informations concernant un état d'utilisation d'un canal de communication pour réaliser la communication.

5. Dispositif de réception (200) de l'appareil endoscopique sans fil selon la revendication 1, dans lequel l'unité de commande (401) est configurée pour détecter un trafic de communication d'un canal de communication appartenant au groupe de canaux de communication qui a été sélectionné en tant qu'état d'utilisation.

6. Dispositif de réception (200) de l'appareil endoscopique sans fil selon la revendication 1, dans lequel l'unité de commande (401) est configurée pour détecter une intensité du signal reçu d'un canal de communication appartenant au groupe de canaux de communication qui a été sélectionné en tant qu'état d'utilisation.

7. Dispositif de réception (200) de l'appareil endoscopique sans fil selon la revendication 5, dans lequel l'unité de commande (401) est configurée pour déterminer si oui ou non il faut réaliser une communication en utilisant un premier canal de communication appartenant au groupe de canaux de communication qui a été sélectionné selon si oui ou non le trafic de communication est inférieur à un seuil prédéterminé.

8. Dispositif de réception (200) de l'appareil endoscopique sans fil selon la revendication 6, dans lequel l'unité de communication (401) est configurée pour déterminer si oui ou non il faut réaliser une communication en utilisant un premier canal de communication appartenant au groupe de canaux de communication qui a été sélectionné selon si oui ou non l'intensité du signal reçu est inférieure à un seuil prédéterminé.

9. Dispositif de réception (200) de l'appareil endoscopique sans fil selon la revendication 1, dans lequel l'unité de mémoire (402) est configurée pour mémoriser un premier canal de communication et un canal de communication adjacent qui appartiennent au même groupe de canaux de communication, dans lequel le chevauchement est égal ou supérieur à une plage prédéterminée.

10. Dispositif de réception (200) de l'appareil endoscopique sans fil selon la revendication 9, dans lequel l'unité de mémoire (402) est configurée pour mémoriser un premier canal de communication et un canal de communication adjacent qui appartiennent à des groupes de canaux de communication différents, le canal de communication adjacent possédant une bande de fréquence d'utilisation ne chevauchant pas celle du premier canal de communication.

11. Procédé de réception pour utilisation dans un dispositif de réception (200) d'un appareil endoscopique sans fil, le procédé comprenant les étapes consistant à :
utiliser une pluralité de canaux de communication, dont chacun possède une bande de fréquence d'utilisation chevauchant partiellement celle d'au moins l'un des autres canaux de communication, dans lequel un groupe de canaux de communication parmi la pluralité de canaux de communication comprend un premier canal de communication utilisé pour connexion logique avec un dispositif de transmission (100) et un canal de communication adjacent possédant une bande de fréquence d'utilisation chevauchant partiellement celle du premier canal de communication ;
communiquer avec le dispositif de transmission (100) en utilisant le premier canal de communication ;
recevoir des données d'image transmises depuis le dispositif de transmission (100) ;
mémoriser une pluralité de groupes de canaux de communication ;
recevoir une sélection d'opérateur d'un numéro de commutateur de canal correspondant à un groupe de canaux de communication arbitraire parmi la pluralité de groupes de canaux de communication qui ont été mémorisés ;
transférer le résultat de sélection vers une unité de commande ;
réaliser, par l'unité de commande, une détection de l'état d'utilisation du premier canal de communication et des canaux de communication adjacents appartenant au groupe de canaux de communication qui a été sélectionné ;
déterminer, par l'unité de commande, si oui ou non il faut réaliser une communication en utilisant le premier canal de communication appartenant au groupe de canaux de communication qui a été sélectionné sur la base des résultats de la détection, déterminer s'il faut ou non réaliser une communication en utilisant le premier canal de communication comprenant les étapes consistant à :
transmettre (S504), par l'unité de communication (404), un paquet de demande de recherche ;
déterminer (S507) si oui ou non un paquet de réponse à la demande de recherche provenant d'un autre terminal de communication sans fil a été reçu ;
exécuter (S508), si le paquet de réponse à la demande de recherche a été reçu, un processus de mise à jour d'un nombre de terminaux de communication sans fil périphériques utilisant le même canal de communication par les étapes consistant à :
- déterminer (S508a) si oui ou non la valeur d'un premier paramètre destiné à mémoriser un ordre de recherche (SCAN_NO) dans une table de recherche correspond à une valeur prédéterminée, dans lequel la valeur prédéterminée étant de 1 correspond au premier canal de communication du groupe de canaux de communication établi par le commutateur de réglage de canal (103) ;
- déterminer (S508b), si la valeur du premier paramètre ne correspond pas à la valeur prédéterminée, si oui ou non un niveau de réception d'une trame du paquet de réponse à la demande de recherche est égal ou supérieur à un niveau prédéterminé ;
- incrémenter (S508c), si le niveau de réception de trame est égal ou supérieur au niveau prédéterminé ou si la valeur du premier paramètre correspond à la valeur prédéterminée, le nombre de terminaux de communication sans fil périphériques utilisant le même canal de communication de 1 ; et
- retourner (S508), après l'opération d'incrémentation ou si le niveau de réception de trame n'est pas égal ou supérieur au niveau prédéterminé, à l'opération consistant à déterminer si oui ou non un paquet de demande de recherche provenant d'un autre dispositif endoscopique sans fil a été reçu ; et
- déterminer (S509), après qu'un temps prédéterminé s'est écoulé, si le nombre de terminaux de communication sans fil périphériques utilisant le même canal de communication est inférieur à un seuil prédéterminé, que le premier canal de communication est disponible.

12. Appareil endoscopique sans fil comprenant le dispositif de réception (200) selon l'une quelconque des revendications 1 à 10 et un dispositif de transmission (100), dans lequel
le dispositif de transmission (100) comprend
une deuxième unité de communication (306) configurée pour communiquer avec le dispositif de réception (200) en utilisant le premier canal de communication et transmettre les données d'image vers le dispositif de réception (200).

13. Programme informatique comprenant des instructions pour mettre en oeuvre les étapes selon la revendication 11 lorsqu'exécuté par l'unité de commande (401) du dispositif de réception (200) d'un appareil endoscopique sans fil selon l'une quelconque des revendications 1 à 10.
